# EUROPEAN PATENT APPLICATION

(11) **EP 4 393 944 A1**
(43) Date of publication of application: **03.07.2024**
(21) Application number: 22866195.5
(22) Date of filing: 20.06.2022
(51) Int. Cl.: C07K 14/78, C07K 1/107, C07K 1/34, A61L 27/24, A61L 27/52

(54) **METHOD FOR PREPARING PEGYLATED COLLAGEN-LIKE PROTEIN AND USE THEREOF**

(30) Priority: 13.09.2021 CN 202111071336
(71) Applicant: Eosvision Medtech Technology Co., Ltd., Suzhou, Jiangsu 215500 (CN)
(72) Inventor: WANG, Chongyu, Suzhou, Jiangsu 215500 (CN); ZHANG, Jiahui, Suzhou, Jiangsu 215500 (CN); ZHANG, Jun, Suzhou, Jiangsu 215500 (CN); LIU, Murong, Suzhou, Jiangsu 215500 (CN); HE, Chaoxian, Suzhou, Jiangsu 215500 (CN)
(74) Representative: Potter Clarkson
(86) International application number: PCT/CN2022/099755
(87) International publication number: WO 2023/035720

(57) **Abstract**

The present application relates to a method for preparing a pegylated collagen-like protein and the use thereof, which belong to the field of biotechnology. Provided in the present application is a method for preparing a pegylated collagen-like protein, the method comprising reacting a collagen-like protein and a polyethylene glycol derivative under the conditions of a pH of 6.0-8.0 and a temperature of 2-8°C to prepare a pegylated collagen-like protein. Comparing other existing methods for preparing the pegylated collagen-like protein, the pegylated collagen-like protein prepared by using the method of the present application has a better cross-linking performance, and after the pegylated collagen-like protein prepared by using the method of the present application is prepared into a hydrogel to form a cornea, the strength can be improved by at least 10 times compared with the pegylated collagen-like protein prepared by means of the existing methods; and in addition, the reaction time for preparing the pegylated collagen-like protein by using the method of the present application is shorter, and the pegylated collagen-like protein capable of being prepared into a hydrogel to form a cornea can be obtained merely by means of reacting for 5-8h.

## Description

### CROSS-REFERENCES TO RELATED APPLICATION

This application claims the priority of Chinese patent application submitted to the China National Intellectual Property Administration of the People's Republic of China on September 13, 2021, with the application number 202111071336.3 and the invention title "METHOD FOR PREPARING PEGYLATED COLLAGEN-LIKE PROTEIN AND USE THEREOF", all of which are incorporated herein by reference.

### FIELD OF THE INVENTION

The present application relates to a method for preparing PEGylated collagen-like protein and its application, and belongs to the field of biotechnology.

### BACKGROUND OF THE INVENTION

The cornea is the convex, highly transparent substance at the front of the eye. It is transversely oval in shape and covers the iris, pupil and anterior chamber, and provides most of the refractive power of the eye. Coupled with the refractive power of the lens, light can be accurately focused on the retina to form an image. The cornea has very sensitive nerve endings. If a foreign object comes into contact with the cornea, the eyelids will involuntarily close to protect the eyes. In order to maintain transparency, the cornea has no blood vessels and obtains nutrients and oxygen through tears and aqueous humor.

The cornea is very fragile. Eye trauma, inflammation, allergic reactions, physical damage, chemical burns, strenuous exercise, overuse of eyes, etc. can all lead to corneal pathology. Once the cornea becomes diseased, it will lead to obvious eye symptoms, such as eye pain, photophobia, tearing, vision loss, etc., and in severe cases, blindness.

Corneal transplantation is a treatment method that replaces the patient's existing diseased cornea with a normal cornea to restore vision in the affected eye or control corneal disease, improve vision, or treat certain corneal diseases. Some corneal diseases that cause severe visual impairment or even blindness in patients can be completely treated through corneal transplantation, helping these unfortunate patients stay away from pain. Because the cornea itself does not contain blood vessels and is in an "immune pardon" status, the success rate of corneal transplantation ranks first among other allogeneic organ transplants.

However, corneal resources are limited and far from meeting the needs of patients. To solve this problem, some researchers proposed artificial keratoplasty. Artificial keratoplasty is a surgical method that uses a special optical device made of transparent medical polymer materials to be implanted into the corneal tissue to replace part of the corneal scar tissue and restore vision. Due to unresolved issues such as the rejection reaction of corneal tissue to synthetic materials, the long-term results are not good, and it often causes aqueous humor leakage at the transplant site and graft shedding, so it is not yet widely used. At this stage, artificial corneas are only suitable for blindness after suffering from various serious corneal diseases, especially those who have all corneal leukoplakia caused by severe chemical burns and who have failed multiple corneal transplants and cannot undergo other surgeries.

On the basis of artificial cornea, some researchers have proposed bionic cornea. For example, in the document "Short peptide analogs as alternatives to collagen in pro-regenerative corneal implants", Jangamreddy, JaganmohanR. et al. proposed a PEGylated collagen-like protein, which was chemically cross-linked to obtain a bionic cornea. This bionic cornea has excellent biocompatibility and can effectively solve the problem of corneal tissue's strong rejection reaction to synthetic materials.

However, the strength of this bionic cornea is very low, only 0.022MPa. If this bionic cornea is used for corneal transplantation, there are still problems such as difficulty in transplantation and the possibility of causing corneal keratoconus.

### SUMMARY OF THE INVENTION

In order to solve the problem of low strength of existing bionic corneas, this application provides a method for preparing a PEGylated collagen-like protein, wherein the method includes the following steps:
a reaction step: reacting a collagen-like protein with a polyethylene glycol derivative at a pH value of 6.0 to 8.0 and a temperature of 2°C to 8°C to obtain a reaction product; wherein the reaction product contains the above PEGylated collagen-like protein.

In one embodiment of the present application, in the reaction step, the molar ratio of the collagen-like protein to the polyethylene glycol derivative is 1 to 16:1.

In one embodiment of the present application, in the reaction step, the molar ratio of the collagen-like protein to the polyethylene glycol derivative is 8 to 12:1.

In one embodiment of the present application, in the reaction step, a reaction solvent of the collagen-like protein and the polyethylene glycol derivative is water or a dilute hydrochloric acid solution.

In one embodiment of the present application, the concentration of the dilute hydrochloric acid solution is 1 mmol/L to 10mmol/L; the pH value of the dilute hydrochloric acid solution is adjusted to 6.0 to 8.0 with a dilute alkali solution.

In one embodiment of the present application, the dilute alkali solution is a sodium hydroxide solution or ammonia water with a pH value of 9.0 to 11.0.

In one embodiment of the present application, in the reaction step, the feed concentration of collagen-like protein in the reaction solvent is 1 mg/mL to 15 mg/mL.

In one embodiment of the present application, in the reaction step, the feed concentration of collagen-like protein in the reaction solvent is 8 mg/mL to 10 mg/mL.

In one embodiment of the present application, after the reaction step, the method further includes a purification step; wherein the purification step is to intercept substances with a molecular weight of greater than or equal to 30,000 Da in the reaction product by filtration to obtain the PEGylated collagen-like protein.

In one embodiment of the present application, the filtration is dialysis or ultrafiltration.

In one embodiment of the present application, the reaction time is 1 hour to 48 hours.

In one embodiment of the present application, the reaction time is 5 hours to 8 hours.

In one embodiment of the present application, the amino acid sequence of the collagen-like protein is as shown in SEQ ID NO. 1

In SEQ ID NO.1, X is 4Hyp (4-hydroxyproline), that is the amino acid sequence of SEQ ID NO.1 is:
H-Pro-Lys-Gly-Pro-Lys-Gly-Pro-Lys-Gly-Pro-Lys-Gly-Pro-Hyp-Gly-Pro-Hyp-Gly-Pro-Hyp-Gly-Pro-Hyp-Gly- Asp-Hyp-Gly-Asp-Hyp-Gly-Asp-Hyp-Gly-Asp-Hyp-Gly-OH.

In one embodiment of the present application, the polyethylene glycol derivative includes one or more of PEG-40k, PEG-20k, PEG-10k or PEG-5k.

In one embodiment of the present application, the number of activating groups of the polyethylene glycol derivative is one or more of 8-am, 4-arm, 2-arm or 1-arm.

In one embodiment of the present application, the number of activating groups of the polyethylene glycol derivative is 4-arm or 8-arm.

In one embodiment of the present application, the activating group of the polyethylene glycol derivative is one or more of -MAL, -NHS, -SG, -SPA, -SS or -EDC.

In one embodiment of the present application, the activating group of the polyethylene glycol derivative is -MAL.

In one embodiment of the present application, one end of the collagen-like protein is connected to a linker; the polyethylene glycol derivative is modified on the linker through an activating group. In one embodiment of the present application, the linker is connected to the N-terminus of the collagen-like protein.

In one embodiment of the present application, the amino acid sequence of the linker is shown in SEQ ID NO. 2 or SEQ ID NO. 3.

In one embodiment of the present application, the amino acid sequence of the linker is shown in SEQ ID NO. 2.

In one embodiment of the present application, the modification site of the polyethylene glycol derivative on the linker is one or more of a thiol group, an amino group, a carboxyl group or an imidazole group.

In one embodiment of the present application, the modification site of the polyethylene glycol derivative on the linker is a thiol group.

In one embodiment of the present application, the core conformation of the PEGylated collagen-like protein is one or more of HG or TP.

In one embodiment of the present application, the core conformation of the polyethylene glycol derivative in the PEGylated collagen-like protein is TP.

In one embodiment of the present application, the amino acid configuration of the collagen-like protein in the PEGylated collagen-like protein is one or more of D-type or L-type.

In one embodiment of the present application, the molecular weight of the PEGylated collagen-like protein is 15,000 to 75,000 Da.

In one embodiment of the present application, the molecular weight of the PEGylated collagen-like protein is 30,000 to 75,000 Da.

The present application also provides a PEGylated collagen-like protein, which is prepared using the above method.

The present application also provides a method for preparing a freeze-dried preparation of PEGylated collagen-like protein, wherein the method includes the following steps:
a freeze-drying step: freeze-drying the PEGylated collagen-like protein prepared using the above method to obtain a freeze-dried preparation.

In one embodiment of the present application, the freeze-drying step is: mixing PEGylated collagen-like protein and a freeze-drying protective agent, and then freeze-drying to obtain a freeze-dried preparation.

In one embodiment of the present application, the freeze-drying protective agent is one or more of mannitol, sucrose or alanine.

In one embodiment of the present application, the freeze-drying includes the following stages:
stage 1: freeze-drying for 6 hours at a temperature of -45°C and a vacuum of 500mTorr;
stage 2: freeze-drying for 17 hours at a temperature of -30°C and a vacuum of 100mTorr; and
stage 3: freeze-drying for 7 hours at a temperature of 25°C and a vacuum of 100 mTorr.

The present application also provides a freeze-dried preparation of PEGylated collagen-like protein, which is prepared using the above method.

This application also provides the application of the PEGylated collagen-like protein prepared by the above method or the freeze-dried preparation prepared by the above method in the manufacture of bionic or regenerative biological material.

In one embodiment of the present application, the bionic or regenerated biological material is cornea.

This application also provides a method for preparing a bionic cornea, wherein the method includes the following steps:
cross-linking step: dissolving the freeze-dried preparation prepared using the above method in a buffer to obtain a solution: mixing the solution and a MPC mother solution to obtain a mixed solution 1; and mixing the mixed solution 1 and a DMTMM mother solution to obtain a mixed solution 2: and
curing step: pouring the mixed solution 2 into the cornea mold and letting it stand to obtain a crude cornea.

In one embodiment of the present application, after the curing step, the method further includes a soaking step; wherein the soaking step is: adding the crude cornea together with the mold into the buffer for a first soaking; after the first soaking is completed, opening the mold for a second soaking; after the second soaking is completed, demoulding, and obtaining a finished cornea.

In one embodiment of the present application, in the cross-linking step, the pH value of the buffer is 5.5 to 8.0.

In one embodiment of the present application, in the cross-linking step, the pH value of the buffer is 6.5 to 7.5.

In one embodiment of the present application, in the cross-linking step, the concentration of the buffer is 0.5 to 0.7mol/L.

In one embodiment of the present application, in the cross-linking step, the buffer is MOPS Buffer, MES Buffer or PBS Buffer.

In one embodiment of the present application, in the cross-linking step, the concentration of PEGylated collagen-like protein in the buffer is 5 g/mL to 40g/mL.

In one embodiment of the present application, in the cross-linking step, the concentration of PEGylated collagen-like protein in the buffer is 12 g/mL to 18g/mL.

In one embodiment of the present application, in the cross-linking step, the concentration of PEGylated collagen-like protein in the buffer is 15g/mL.

In one embodiment of the present application, in the cross-linking step, the mixing mass ratio of the dissolving solution and the MPC mother solution is 2:1 to 4:1.

In one embodiment of the present application, in the cross-linking step, the mixing mass ratio of the mixed solution 1 and the DMTMM mother solution is 5:1 to 7:1.

In one embodiment of the present application, the cross-linking step is completed at a temperature of 25°C to 60°C.

In one embodiment of the present application, the cross-linking step is completed at a temperature of 45°C to 55°C.

In one embodiment of the present application, in the curing step, the standing is performed at a temperature of 4°C to 35°C.

In one embodiment of the present application, in the curing step, the standing is performed at a temperature of 4°C to 25°C.

In one embodiment of the present application, in the curing step, the standing is performed for 8 hours to 20 hours.

In one embodiment of the present application, in the soaking step, the pH value of the buffer is 5.5 to 8.0.

In one embodiment of the present application, in the soaking step, the pH value of the buffer is 6.5 to 7.5.

In one embodiment of the present application, in the soaking step, the concentration of the buffer solution is 0.05 mol/L to 1 mol/L.

In one embodiment of the present application, in the soaking step, the buffer is MOPS Buffer, MES Buffer or PBS Buffer.

In one embodiment of the present application, the first soaking is performed at a temperature of 4°C to 35°C for 5 hours to 24 hours.

In an embodiment of the present application, the second soaking is performed at a temperature of 4°C to 35°C for 3 hours to 10 hours.

In one embodiment of the present application, the components of the MPC mother solution include MPC (2-methacryloyloxyethylphosphocholine), PEGDA(poly(ethylene glycol) diacrylate), TEMED (N, N, N', N'-Tetramethylethylenediamine) and a solvent.

In one embodiment of the present application, in the MPC mother solution, the concentration of MPC is 15 g/mL to 40g/mL: in terms of volume percentage, the concentration of PEGDA in the MPC mother solution is 0.6% to 15%, and the concentration of TEMED is 0.05% to 2%.

In one embodiment of the present application, the concentration of PEGDA in the MPC mother solution is 8% to 12%.

In one embodiment of the present application, the concentration of PEGDA in the MPC mother solution is 10%.

In one embodiment of the present application, the solvent of the MPC mother solution is a buffer; the pH value of the buffer is 6.0 to 8.0.

In one embodiment of the present application, in the MPC mother solution, the pH value of the buffer is 6.5 to 7.5.

In one embodiment of the present application, in the MPC mother solution, the concentration of the buffer solution is 0.5 mol/L to 0.7 mol/L.

In one embodiment of the present application, in the MPC mother solution, the buffer is MOPS Buffer, MES Buffer or PBS Buffer

In one embodiment of the present application, the components of the DMTMM mother solution include DMTMM (4-(4,6-dimethoxytriazin-2-yl)-4-methylmorpholine hydrochloride), APS (ammonium persulfate) and a solvent.

In one embodiment of the present application, in the DMTMM mother solution, the concentration of DMTMM is 5 g/mL to 20g/mL, and the concentration of APS is 0.5 g/mL to 5g/mL.

In one embodiment of the present application, the solvent of the DMTMM mother solution is a buffer; the pH value of the buffer is 6.0 to 8.0.

In one embodiment of the present application, in the DMTMM mother solution, the pH value of the buffer is 6.5 to 7.5.

In one embodiment of the present application, in the DMTMM mother solution, the concentration of the buffer is 0.5 mol/L to 0.7 mol/L.

In one embodiment of the present application, in the DMTMM mother solution, the buffer is MOPS Buffer, MES Buffer PBS Buffer

This application also provides a bionic cornea, which is prepared using the above method.

The technical solution of this application has the following advantages:
1. This application provides a method for preparing PEGylated collagen-like proteins. In the method, collagen-like proteins and polyethylene glycol derivatives are reacted at a pH value of 6.0 to 8.0 and a temperature of 2°C to 8°C to prepare PEGylated collagen-like proteins. Compared with other existing methods for preparing PEGylated collagen-like proteins, the PEGylated collagen-like proteins prepared using the method of the present application have better cross-linking properties. After the PEGylated collagen-like protein prepared using the method of the present application is made into a hydrogel and formed into the cornea, the strength can be increased by more than 10 times compared with the PEGylated collagen-like protein prepared using the existing method (The strength of the cornea made from PEGylated collagen-like proteins in the literature "Short peptide analogs as alternatives to collagen in pro-regenerative corneal implants" is only 0.022MPa).

Moreover, compared with other existing methods for preparing PEGylated collagen-like proteins, the reaction time for preparing PEGylated collagen-like proteins using the method of the present application is shorter, and it only takes 5 hours to 8 hours to react to obtain the PEGylated collagen-like proteins that can be made into hydrogels to form corneas (In the literature "Short peptide analogs as alternatives to collagen in pro-regenerative corneal implants"), it took a reaction time of 4 weeks to prepare the PEGylated collagen-like proteins that can be made into hydrogels to form corneas).

Further, in the reaction step, the molar ratio of the collagen-like protein and the polyethylene glycol derivative is 8 to 12:1; the cross-linking properties of the PEGylated collagen-like protein prepared under this molar ratio is better.

Further, in the reaction step, the feed concentration of collagen-like protein in the reaction solvent is 8 mg/mL to 10 mg/mL; the cross-linking properties of the PEGylated collagen-like protein prepared at this feed concentration is better.

In summary, the method of the present application and the PEGylated collagen-like protein prepared using the method of the present application have extremely high application prospects in the preparation of bionic or regenerative biological materials such as bionic corneas.

2. This application provides a method for preparing a freeze-dried preparation of PEGylated collagen-like protein. The method is to mix the PEGylated collagen-like protein prepared by the above method and a freeze-drying protective agent and then freeze-dry to prepare a freeze-dried preparation of PEGylated collagen-like protein. The freeze-drying protective agent can protect the PEGylated collagen-like protein prepared using the above method, so that the structure of the PEGylated collagen-like protein prepared using the above method will not be destroyed during the freeze-drying process, so that after the freeze-dried preparation prepared by the method of the present application is made into a hydrogel to form a cornea, the strength is further improved.

In summary, the method of the present application and the freeze-dried preparation prepared using the method of the present application have extremely high application prospects in the preparation of bionic or regenerative biological materials such as bionic corneas.

3. This application provides a method for preparing bionic cornea. The method is to first dissolve the freeze-dried preparation prepared using the above method in MES Buffer to obtain a dissolving solution, and then mix the dissolving solution and MPC mother solution to obtain a mixed solution 1, and then the mixed solution 1 and DMTMM mother solution are mixed to obtain a mixed solution 2. Finally, the mixed solution 2 is poured into a cornea mold and left to stand to obtain a crude cornea. Compared with other existing methods of preparing bionic corneas, the strength of the cornea prepared using the method of the present application is high, and can be improved by more than 10 times compared with the cornea prepared using other existing methods of preparing bionic corneas (The strength of the cornea in the literature "Short peptide analogs as alternatives to collagen in pro-regenerative corneal implants" is only 0.022MPa).

Further, in the method, the freeze-dried preparation is cross-linked under the conditions of pH value of 6.5 to 7.5 and temperature of 45°C to 55°C; the bionic cornea produced under this cross-linking condition has higher strength.

Further, in the method, the concentration of PEGylated collagen-like protein is controlled at 12 g/mL to 18g/mL; the bionic cornea produced at this concentration of PEGylated collagen-like protein is stronger.

Further, in the method, the concentration of PEGDA in the MPC mother solution is controlled at 8% to 12%; the bionic cornea produced at this concentration of PEGDA is stronger.

### DETAILED DESCRIPTION

The following examples are provided to better understand the present application. They are not limited to the best implementation mode, and do not limit the content and protection scope of the present application. Any product that is identical or similar to the present application, which is drived by any person under the inspiration of the present application or by combining the present application with other features of the prior art, falls within the protection scope of the present application.

If no specific experimental steps or conditions are specified in the following examples, the operations or conditions can be carried out according to the conventional experimental steps described in literature in the field. If the manufacturer of the reagents or instruments used is not indicated, they are all conventional reagent products that can be purchased commercially. In the following examples, the synthesis of collagen-like proteins and the connection between collagen-like proteins and linkers were completed by Shanghai Ambio Pharm.InC. The polyethylene glycol derivatives in the following examples were purchased from Xiamen Sanobange Biotechnology Co., Ltd.

### Example 1-1: A method for preparing PEGylated collagen-like protein

This example provides a method for preparing PEGylated collagen-like protein, wherein the method includes the following steps:
a reaction step: the collagen-like protein (the amino acid sequence of the collagen-like protein is as shown in SEQ ID NO. 1, and the N-terminal of the collagen-like protein is connected with a linker with the amino acid sequence as shown in SEQ ID NO. 2) and the polyethylene glycol derivative (8-arm-PEG-40k-MAL) was dissolved in a dilute hydrochloric acid solution with a concentration of 5mmol/L (the pH value of the dilute hydrochloric acid solution was adjusted to 6.5 with sodium hydroxide solution of pH value of 11.0), so that in the dilute hydrochloric acid solution, the molar concentrations of collagen-like protein and polyethylene glycol derivative were 2.4mmol/L and 0.3mmol/L respectively to obtain a reaction system; the reaction system was reacted at a pH value of 6.5 and a temperature of 5°C for 12 hours to obtain the reaction product;
a purification step: the reaction product is ultrafiltrated through an ultrafiltration membrane with a pore size of 30,000 Da at 5°C to intercept substances with a molecular weight of 30,000 Da or greater in the reaction product to obtain PEGylated collagen-like protein 1.

### Example 2-1: A method for preparing a freeze-dried preparation of PEGylated collagen-like protein

This example provides a method for preparing a freeze-dried preparation of PEGylated collagen-like protein, wherein the method includes the following steps:
mixing the PEGylated collagen-like protein 1 prepared in Example 1 and mannitol in a mass ratio of 1:5 to obtain a freeze-dried system; and freeze-drying the freeze-dried system to obtain a freeze-dried preparation 1.

Among them, freeze-drying includes the following stages:
stage 1: freeze-drying for 6 hours at a temperature of -45°C and a vacuum of 500mTorr:
stage 2: freeze-drying for 17 hours at a temperature of -30°C and a vacuum of 100mTorr; and
stage 3: freeze-drying for 7 hours at a temperature of 25°C and a vacuum of 100 mTorr.

### Example 1-2: A method for preparing PEGylated collagen-like protein

This example provides a method for preparing PEGylated collagen-like protein. The method is based on the method of Example 1-1, and the reaction temperature (5°C) is replaced by 2°C, 8°C, 25°C, and 37°C, respectively, to prepare PEGylated collagen-like proteins 2 to 5.

### Example 2-2: A method for preparing a freeze-dried preparation of PEGylated collagen-like protein

This example provides a method for preparing a freeze-dried preparation of PEGylated collagen-like protein. The method is based on the method of Example 2-1, and the PEGylated collagen-like protein 1 is replaced with the PEGylated collagen-like proteins 2 to 5 prepared in Example 1-2, respectively, to prepare freeze-dried preparations 2 to 5.

### Example 1-3: A method for preparing PEGylated collagen-like protein

This example provides a method for preparing PEGylated collagen-like protein. The method is based on the method of Example 1-1, and the pH value (6.5) of the reaction system is replaced with pH 2.5, pH 4.5, pH 5.5, pH 6.0, pH 7.0, pH 8.0, pH 8.5, and pH 10.5, respectively, to prepare PEGylated collagen-like proteins 6 to 13.

### Example 2-3: A method for preparing a freeze-dried preparation of PEGylated collagen-like protein

This example provides a method for preparing a freeze-dried preparation of PEGylated collagen-like protein. The method is based on the method of Example 2-1, and the PEGylated collagen-like protein 1 is replaced with PEGylated collagen-like proteins 6 to 13 prepared in Example 1-3, respectively, to prepare freeze-dried preparations 6 to 13.

### Example 1-4: A method for preparing PEGylated collagen-like protein

This example provides a method for preparing PEGylated collagen-like proteins. The method is based on the method of Example 1-1 by replacing the molar concentrations of the collagen-like proteins and polyethylene glycol derivatives in a dilute hydrochloric acid solution, respectively:
the molar concentrations of collagen-like protein and polyethylene glycol derivative in dilute hydrochloric acid solution are 3.0mmol/L and 0.375mmol/L respectively;
the molar concentrations of collagen-like protein and polyethylene glycol derivative in dilute hydrochloric acid solution are 1.8mmol/L and 0.225mmol/L respectively; and
the molar concentrations of collagen-like protein and polyethylene glycol derivative in dilute hydrochloric acid solution are 1.2mmol/L and 0.15mmol/L respectively,
to prepare PEGylated collagen-like proteins 14 to 16.

### Example 2-4: A method for preparing a freeze-dried preparation of PEGylated collagen-like protein

This example provides a method for preparing a freeze-dried preparation of PEGylated collagen-like protein. The method is based on the method of Example 2-1, and the PEGylated collagen-like protein 1 is replaced with the PEGylated collagen-like proteins 14 to 16 prepared in Example 1-4, respectively, to prepare freeze-dried preparations 14 to 16.

### Example 1-5: A method for preparing pegylated collagen-like protein

This example provides a method for preparing PEGylated collagen-like protein. The method is based on the method of Example 1-1, and the reaction time (12h) is replaced by 4h, 8h, 16h, 24h respectively, to prepare PEGylated collagen-like proteins 17 to 20.

### Example 2-5: A method for preparing a freeze-dried preparation of PEGylated collagen-like protein

This example provides a method for preparing a freeze-dried preparation of PEGylated collagen-like protein. The method is based on the method of Example 2-1, and the PEGylated collagen-like protein 1 is replaced with the PEGylated collagen-like proteins 17 to 20 prepared in Example 1-5, respectively, to prepare freeze-dried preparations 17 to 20.

### Example 2-6: A method for preparing a freeze-dried preparation of PEGylated collagen-like protein

This example provides a method for preparing a freeze-dried preparation of PEGylated collagen-like protein. The method is based on the method of Example 2-1 by replacing the freeze-drying protective agent (mannitol) with sucrose, and alanine, respectively, to prepare the freeze-dried preparations 21 and 22.

### Experimental Example 1: Experiment on the influence of preparation technology on the cross-linking properties of PEGylated collagen-like proteins and their freeze-dried preparations

This experimental example provides an experiment on the influence of the preparation process on the cross-linking properties of PEGylated collagen-like proteins and their freeze-dried preparations. The experimental process is as follows:
Referring to the method for preparing the freeze-dried preparation 1, the collagen-like protein with the amino acid sequence shown in SEQ ID NO. 1 is directly prepared into a freeze-dried preparation. This freeze-dried preparation is used as a blank control. The freeze-dried preparations 1-22 prepared in Examples 2-1 to 2-6 were respectively made into corneas to obtain corneas 1 to 22. Use a universal tensile machine to detect the strength of corneas 1 to 22. The test results are shown in Table 1.

Among them, the cornea preparation process is as follows:
Cross-linking step: the freeze-dried preparation was dissolved in MES Buffer with a concentration of 0.5 mol/L and pH value of 5.5, so that the concentration of PEGylated collagen-like protein in the MES Buffer was 12.5g/mL to obtain a dissolved solution; the dissolving solution and MPC mother solution were mixed in a mass ratio of 5:1 to obtain mixed solution 1; mixed solution 1 and DMTMM mother solution were mixed in a mass ratio of 7:1 to obtain mixed solution 2. The entire cross-linking process was completed at 45°C;
Curing step: the mixed solution 2 was poured into the cornea mold and place it at 25°C for 12 hours to obtain the crude cornea product;
Soaking step: the crude cornea product (with the mold) was added to PBS Buffer with a concentration of 0.1 mol/L and pH value of 6.5. After soaking at 4°C for 24h, open the mold, continue to soak at 4°C for 4h, demould, and obtain the finished cornea.

Among them, the formula of MPC mother solution is: 30 g/mL MPC, 10% PEGDA (v/v), 1% TEMED (v/v), the solvent is 0.5mol/L of MOPS Buffer with pH value of 5.5;
The formula of DMTMM mother solution is: 10g/mL DMTMM, 15g/mL APS, solvent is 0.5mol/Lof MOPS Buffer with pH value of 5.5.

It can be seen from Table 1 that the preparation process will affect the cross-linking properties of PEGylated collagen-like proteins and their freeze-dried preparations, which in turn will affect the hydrogels formed into cornea. Moreover, the freeze-dried excipients provide skeleton support and protection to PEGylated collagen-like protein, thereby affecting the spatial conformation and biological activity of the polymer, resulting in differences in hardness after film formation. The corneas 1 to 22 prepared by using the freeze-dried preparations 1 to 22 in Examples 2-1 to 2-6 have better strength, and at the same time the biocompatibility of the product is improved. In bionic or regenerative organisms such as bionic corneas, it has extremely high application prospects in the preparation of materials.

**Table 1 the strength of corneas 1 to 22**

| Group | Strength (MPa) | Group | Strength (MPa) |
|---|---|---|---|
| Cornea 1 | 0.083 | Cornea 13 | (No film formation) |
| Cornea 2 | 0.080 | Cornea 14 | 0.076 |
| Cornea 3 | 0.075 | Cornea 15 | 0.067 |
| Cornea 4 | 0.025 | Cornea 16 | 0.052 |
| Cornea 5 | / (No film formation) | Cornea 17 | 0.016 |
| Cornea 6 | / (No film formation) | Cornea 18 | 0.037 |
| Cornea 7 | 0.019 | Cornea 19 | 0.084 |
| Cornea 8 | 0.036 | Cornea 20 | 0.082 |
| Cornea 9 | 0.074 | Cornea 21 | 0.061 |
| Cornea 10 | 0.072 | Cornea 22 | 0.047 |
| Cornea 11 | 0.062 | Cornea ( blank control) | 0.014 |
| Cornea 12 | 0.035 | | |

### Example 3-1: A method of preparing bionic cornea

This example provides a method for preparing bionic cornea, wherein the method includes the following steps:
Cross-linking step: the freeze-dried preparation prepared in Example 2-1 was dissolved in MES Buffer with a concentration of 0.5 mol/L and pH value of 6.5, so that the concentration of PEGylated collagen-like protein in the MESB buffer was 15 g/mL, to obtain the dissolving solution; the dissolving solution and the MPC mother solution were mixed according to the mass ratio of 4:1 to obtain the mixed solution 1; the mixed solution 1 and the DMTMM mother solution were mixed according to the mass ratio 7:1 to obtain the mixed solution 2. The entire cross-linking process was completed at 45°C;
Curing step: the mixed solution 2 was poured into the cornea mold and placed it at 25°C for 12 hours to obtain the crude cornea;
Soaking step: the crude cornea (with the mold) was added into PBS Buffer with a concentration of 0.1 mol/L and pH value of 6.5, soak it at 4°C for 24 hours, open the mold, continue soaking at 4°C for 4 hours, demould, and obtain the bionic Cornea 23.

Among them, the formula of MPC mother solution is: 30% MPC (w/v, g/mL), 10% PEGDA (v/v), 1% TEMED (v/v), the solvent is 0.5mol/L of MES Buffer with pH value of 6.5.

The formula of DMTMM mother solution is: 10% DMTMM (w/v, g/mL), 15% APS (w/v, g/mL), and the solvent is 0.5 mol/L of MES Buffer with pH value of 6.5.

### Example 3-2: A method of preparing bionic cornea

This example provides a method for preparing bionic cornea. The method is based on the method of Example 3-1 and replaces the cross-linking temperature (45°C) with 20°C, 25°C, 40°C, 50°C, 55°C, 60°C, 65°C, respectively, to prepare bionic corneas 24 to 30.

### Example 3-3: A method of preparing bionic cornea

This example provides a method for preparing bionic cornea. The method is based on the method of Example 3-1 and replaces the pH value (6.5) of the Buffer with 5, 5.5, 6, 7, 7.5, 8, 8.5, 9, respectively, to prepare bionic corneas 31 to 38.

### Example 3-4: A method of preparing bionic cornea

This example provides a method for preparing bionic cornea. The method is based on the method of Example 3-1, and the concentration of PEGylated collagen-like protein in MES Buffer (15g/mL) is replaced by 1g/mL, 5g/mL, 10g/mL, 20g/mL, 25g/mL, 30g/mL, 35g/mL, 40g/mL, 45g/mL, respectively, to prepare bionic corneas 39 to 47.

### Example 3-5: A method of preparing bionic cornea

This example provides a method for preparing bionic cornea. The method is based on the method of Example 3-1 and replaces the concentration of PEGDA (10%, v/v) in the MPC mother solution with 0.5%, 0.6%, 1%, 5%, 8%, 12%, 15% (v/v), respectively, to prepare bionic corneas 48 to 54.

### Experimental Example 2: Experiment on the influence of preparation technology on corneal strength properties

This experimental example provides an experiment on the impact of the preparation process on the strength properties of the cornea. The experimental process is as follows:
Use a universal tensile machine to detect the strength of corneas 23 to 54. The test results are shown in Table 2.

As can be seen from Table 2, the preparation process will affect the strength properties of the cornea. Among them, if the concentration of PEGylated collagen-like protein is too low, the strength of the cornea will deteriorate, and if the concentration is too high, the curing time of the cornea will be extremely short, making it impossible to complete the following casting step. Since the isoelectric point of PEGylated collagen-like protein is neutral, when the cross-linking pH is far away from the isoelectric point, the groups participating in the reaction cannot be close to each other, resulting in low corneal strength or no gelation of PEGylated collagen-like protein. The hydrogel made of PEGylated collagen-like protein is a temperature-sensitive hydrogel. When the temperature is low, its solubility is not good, which will cause the concentration of corneal solids to be low and the strength to change small, high temperature will cause its cross-linking speed to be extremely fast, which will cause it to be unable to be poured into the mold and gel in advance. Too low a concentration of PEGDA in the MPC mother solution will cause the corneal strength to deteriorate, and too high a concentration will cause excessive cross-linking of PEGylated collagen-like protein, which makes the hydrogel made of PEGylated collagen-like protein more brittle and reduces its toughness, thereby worsening the strength of the cornea. In Table 1, corneas 23, 25 to 29, 32to 36, 40 to 45 and 50 to 54 all have better strength and good biocompatibility, and have great application prospects in the field of corneal transplantation.

**Table 2 the strength of Corneas 23 to 54**

| Group | Strength (MPa) | Group | Strength (MPa) |
|---|---|---|---|
| Cornea 23 | 0.271 | Cornea 39 | / (No film formation) |
| Cornea 24 | / (No film formation) | Cornea 40 | 0.097 |
| Cornea 25 | 0.093 | Cornea 41 | 0.125 |
| Cornea 26 | 0.201 | Cornea 42 | 0.232 |
| Cornea 27 | 0.255 | Cornea 43 | 0.213 |
| Cornea 28 | 0.196 | Cornea 44 | 0.179 |
| Cornea 29 | 0.154 | Cornea 45 | 0.163 |
| Cornea 30 | / (No film formation) | Cornea 46 | / (No film formation) |
| Cornea 31 | 0.046 | Cornea 47 | / (No film formation) |
| Cornea 32 | 0.082 | Cornea 48 | 0.024 |
| Cornea 33 | 0.169 | Cornea 49 | 0.037 |
| Cornea 34 | 0.213 | Cornea 50 | 0.109 |
| Cornea 35 | 0.201 | Cornea 51 | 0.143 |
| Cornea 36 | 0.133 | Cornea 52 | 0.206 |
| Cornea 37 | / (No film formation) | Cornea 53 | 0.211 |
| Cornea 38 | / (No film formation) | Cornea 54 | 0.213 |

Obviously, the above-mentioned examples are only examples for clear explanation and are not intended to limit the implementation. For those of ordinary skill in the art, other different forms of changes or modifications can be made based on the above description. An exhaustive list of all implementations is neither necessary nor possible. The obvious changes or modifications derived therefrom are still within the scope of protection created by this application.

## Claims

1. A method for preparing a PEGylated collagen-like protein, wherein the method includes the following steps:
a reaction step: reacting a collagen-like protein with a polyethylene glycol derivative at a pH value of 6.0 to 8.0 and a temperature of 2°C to 8°C to obtain a reaction product; wherein the reaction product contains the above PEGylated collagen-like protein.

2. The method of claim 1, wherein in the reaction step, the molar ratio of the collagen-like protein to the polyethylene glycol derivative is 1 to 16:1.

3. The method of claim 2, wherein in the reaction step, the molar ratio of the collagen-like protein to the polyethylene glycol derivative is 8 to 12:1.

4. The method of any one of claims 1 to 3, wherein in the reaction step, a reaction solvent of the collagen-like protein and the polyethylene glycol derivative is water or a dilute hydrochloric acid solution.

5. The method of claim 4, wherein the concentration of the dilute hydrochloric acid solution is 1 mmol/L to 10 mmol/L; the pH value of the dilute hydrochloric acid solution is adjusted to 6.0 to 8.0 with a dilute alkali solution.

6. The method of claim 5, wherein the dilute alkali solution is a sodium hydroxide solution or ammonia water with a pH value of 9.0 to 11.0.

7. The method of any one of claims 1 to 3, wherein in the reaction step, the feed concentration of the collagen-like protein in the reaction solvent is 1 mg/mL to 15 mg/mL.

8. The method of claim 7, wherein in the reaction step, the feed concentration of the collagen-like protein in the reaction solvent is 8 mg/mL to 10 mg/mL.

9. The method of any one of claims 1 to 3, wherein after the reaction step, the method further comprises a purification step; wherein the purification step is to intercept substances with a molecular weight greater than or equal to 30,000 Da in the reaction product by filtration to obtain the PEGylated collagen-like protein.

10. The method of claim 9, wherein the filtration is dialysis or ultrafiltration.

11. The method of any one of claims 1 to 3, wherein the amino acid sequence of the collagen-like protein is as shown in SEQ ID NO. 1.

12. A PEGylated collagen-like protein, wherein the PEGylated collagen-like protein is prepared using the method of any one of claims 1 to 11.

13. A method for preparing a freeze-dried preparation of pegylated collagen-like protein, wherein the method includes the following steps:
a freeze-drying step: freeze-drying the PEGylated collagen-like protein prepared using the method of any one of claims 1 to 11 to obtain a freeze-dried preparation.

14. The method of claim 13, wherein the freeze-drying step is: mixing PEGylated collagen-like protein and a freeze-drying protective agent, and then freeze-drying to obtain a freeze-dried preparation.

15. The method of claim 14, wherein the freeze-drying protective agent is one or more of mannitol, sucrose or alanine.

16. The method of any one of claims 13 to 15, wherein the freeze-drying includes the following stages:
stage 1: freeze-drying for 6 hours at a temperature of -45°C and a vacuum of 500mTorr;
stage 2: freeze-drying for 17 hours at a temperature of -30°C and a vacuum of 100mTor; and
stage 3: freeze-drying for 7 hours at a temperature of 25°C and a vacuum of 100 mTom.

17. A freeze-dried preparation of PEGylated collagen-like protein, wherein the freeze-dried preparation is prepared using the method of any one of claims 13 to 16.

18. Application of the PEGylated collagen-like protein prepared by the method of any one of claims 1 to 11 or the PEGylated collagen-like protein of claim 12 or the freeze-dried preparation prepared by the method of any one of claims 13 to 16 or the freeze-dried preparation of claim 17 in the manufacture of bionic or regenerative biological material.

19. The application of claim 18, wherein the bionic or regenerative biological material is cornea.

20. A method for preparing a bionic cornea, wherein the method includes the following steps:
cross-linking step: dissolving the freeze-dried preparation prepared using the method of any one of claims 13 to 16 in a buffer to obtain a dissolving solution; mixing the dissolving solution and a MPC mother solution to obtain a mixed solution 1; and mixing the mixed solution and a DMTMM mother solution to obtain a mixed solution 2; and
curing step: pouring the mixed solution 2 into the cornea mold and letting it stand to obtain a crude cornea.

21. The method of claim 20, wherein after the curing step, the method further includes a soaking step; wherein the soaking step is: adding the crude cornea together with the mold into the buffer for a first soaking, after the first soaking is completed, opening the mold for a second soaking, after the second soaking is completed, demolding, and obtaining a finished cornea.

22. The method of claim 20 or 21, wherein in the cross-linking step, the pH value of the buffer is 5.5 to 8.0.

23. The method of claim 20 or 21, wherein in the cross-linking step, the concentration of PEGylated collagen-like protein in the buffer is 5 g/mL to 40 g/mL.

24. The method of claim 20 or 21, wherein in the cross-linking step, the mixing mass ratio of the dissolving solution and the MPC mother solution is 2:1 to 4:1.

25. The method of claim 20 or 21, wherein in the cross-linking step, the mixing mass ratio of the mixed solution 1 and the DMTMM mother solution is 5:1 to 7:1.

26. The method of claim 20 or 21, wherein the cross-linking step is completed at a temperature of 25°C to 60°C.

27. The method of claim 20 or 21, wherein in the curing step, the standing is performed at a temperature of 4°C to 35°C.

28. The method of claim 20 or 21, wherein in the soaking step, the pH value of the buffer is 5.5 to 8.0.

29. The method of claim 21, wherein the first soaking is performed at a temperature of 4°C to 35°C for 5 hours to 24 hours.

30. The method of claim 21, wherein the second soaking is performed at a temperature of 4°C to 35°C for 3 hours to 10 hours.

31. The method of claim 20 or 21, wherein the components of the MPC mother solution include MPC, PEGDA, TEMED and a solvent.

32. The method of claim 31, wherein in the MPC mother solution, the concentration of MPC is 15 g/mL to 40g/mL; in terms of volume percentage, in the MPC mother solution, the concentration of PEGDA is 0.6% to 15%, the concentration of TEMED is 0.05% to 2%.

33. The method of claim 31, wherein the solvent of the MPC mother solution is a buffer; the pH value of the buffer is 6 to 8.

34. The method of claim 20 or 21, wherein the components of the DMTMM mother solution include DMTMM, APS and a solvent.

35. The method of claim 34, wherein in the DMTMM mother solution, the concentration of DMTMM is 5 g/mL to20g/mL, and the concentration of APS is 0.5 g/mL to 5g/mL.

36. The method of claim 34, wherein the solvent of the DMTMM mother solution is a buffer; the pH value of the buffer is 6.0 to 8.0.

37. A bionic cornea, wherein the bionic cornea is prepared using the method of any one of claims 20 to 36.
